Europäisches Patentamt

European Patent Office

Office européen des brevets

(11) Publication number: **0 425 902 A1**

# EUROPEAN PATENT APPLICATION

(21) Application number: 90119954.7

(22) Date of filing: 18.10.90

(51) Int. Cl.⁵: **A61K 31/34**

(30) Priority: 25.10.89 IT 2212989

(43) Date of publication of application:
08.05.91 Bulletin 91/19

(84) Designated Contracting States:
AT BE CH DE DK ES FR GB GR IT LI LU NL SE

(71) Applicant: ISCOFAR Sas di Paolo E. Ghirardi
Corso Venezia 16
I-20121 Milano(IT)

(72) Inventor: Ghirardi, E. Paolo
Via Podgora 1
I-20122 Milan(IT)
Inventor: Dei Cas, Livio
Via Nazionale 16
I-23032 Bormio (Sondrio)(IT)

(74) Representative: Gervasi, Gemma
NOTARBARTOLO & GERVASI Srl Viale
Bianca Maria 33
I-20122 Milan(IT)

(54) Use of isosorbide 2-mononitrate in the preparation of pharmaceutical compositions for treating angina pectoris.

㊐ Isosorbide 2-mononitrate for preparing pharmaceutical compositions in a form suitable for the chronic treatment of angina pectoris and in a form suitable for the acute treatment of angina pectoris.

EP 0 425 902 A1

# USE OF ISOSORBIDE 2-MONONITRATE IN THE PREPARATION OF PHARMACEUTICAL COMPOSITIONS FOR TREATING ANGINA PECTORIS

## Field of the invention

This invention relates to pharmaceutical compositions of isosorbide 2-mononitrate for treating angina pectoris.

## Prior art

It is well known to use organic nitroderivatives in treating the main cardiopathies.

Said nitroderivatives represent the drugs of primary use in the treatment of angina pectoris, and also play an important role in the vasodilative treatment of cardiac insufficiency and as agents able to reduce the infarcted area in acute myocardial infarct.

The main nitroderivatives currently used in therapy are nitroglycerin, which was the first used in order of time, pentaerythritol tetranitrate, isosorbide dinitrate and isosorbide 5-mononitrate.

These compounds have considerable therapeutic effectiveness when administered in acute form, but have the drawback that when administered in chronic form they rapidly lose their activity because tolerance develops.

There therefore still remains the problem of providing a drug for use in chronic form in the treatment of cardiac affections, particularly in the treatment of angina pectoris, which does not lose its efficiency with time.

Even the acute therapy of angina pectoris as currently carried out is not totally satisfactory.

With nitroglycerin, which is the most widely used drug, there is rapid initiation of activity, but this rapidly falls off.

Isosorbide dinitrate does not act as such but via the prevalent 5-nitroderivative and 2-nitroderivative metabolite. Apart from the fact that such an action mechanism inevitably results in a considerable induction time (not welcome when treating an acute form), it is also true that the active metabolite, isosorbide 5-mononitrate, has a lengthy action time and persistent hematic levels which rapidly induce tolerance by saturating the receptors. In this respect, isosorbide 5-mononitrate as such is no longer habitually used in the treatment of acute forms.

## Summary

It has now been found that the problems of the prior art are solved by the use of isosorbide 2-mononitrate which, totally unexpectedly, has pharmacological characteristics which differ from the nitroderivatives of the prior art, particularly with regard to tolerance, which develops much more slowly for this drug.

All nitroderivatives induce a tolerance phenomenon but the degree of the lack of the vasodilatory effect was lesser in the case of isosorbide 2-nitroderivative. In order to prove this behaviour, the vasodilatory activity of isosorbide 2-nitroderivative (in the following indicated as 2MN) was evaluated on different isolated anatomical preparations in comparison with nitroglycerin (NTG), isosorbide dinitrate (ISDN) and isosorbide 5-nitroderivative (5MN). Isolated rabbit hearts and aortic strips were used as experimental model to evaluate drugs acting on coronary resistance and vascular contractility.

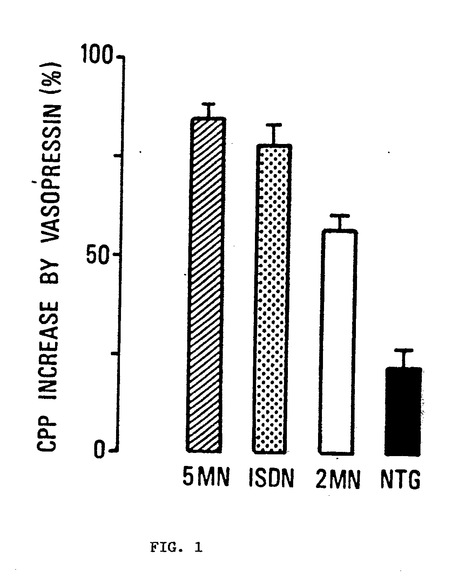

Vasodilatory effect was evaluated on isolated rabbit heart in presence of vasopressin-induced spasm by using concentration of $10^{-5}$ M of the tested compounds. The effects obtained in reducing the "coronary perfusion pressure" (CPP) are reported in Fig. 1. It is evident that NTG is the more active compound being able to reduce coronary spasm of 80 % while 2MM was actually more active than 5MN and ISDN.

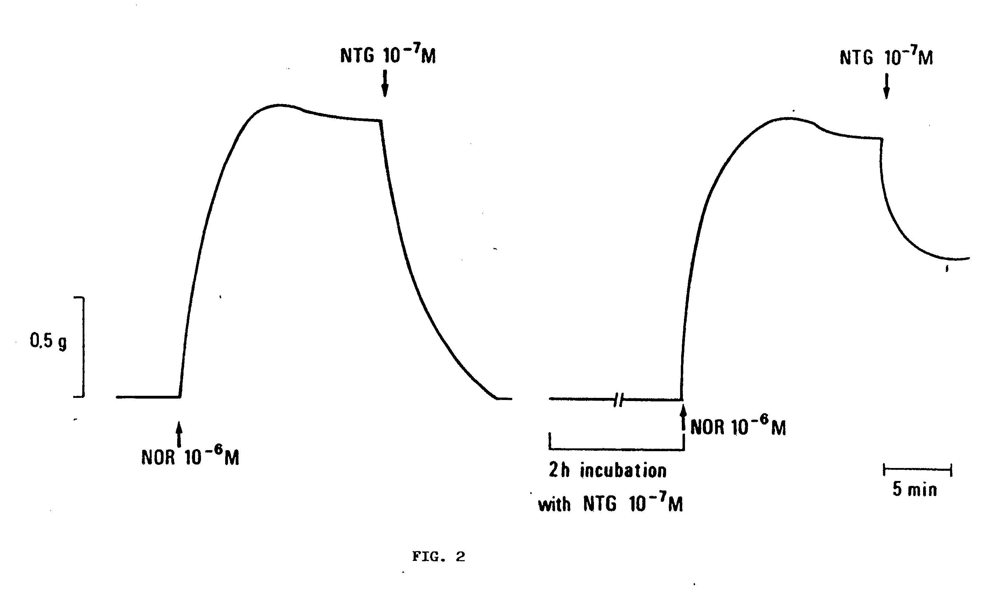

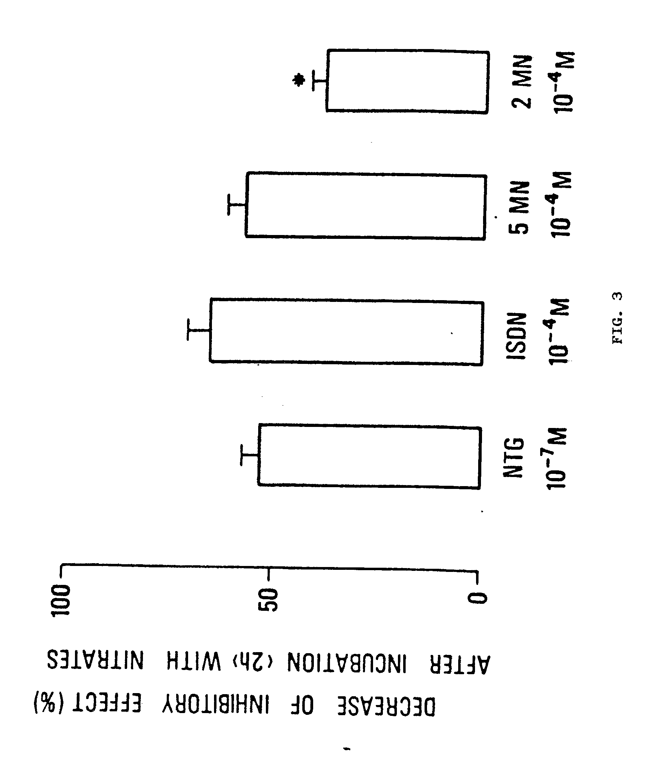

Induction of nitrate tolerance elicited a loss of activity of the all nitroderivatives, as it is represented for NTG in Fig. 2 which shows nitroglycerin (NTG)-induced tolerance in rabbit aortic strips preineubated with the same compound for two hours. However, the degree of such desensitization was significantly lower in the case of 2MN as shown by Fig. 3 wherein percentage values of the inhibitory activity are reported for NTG, ISDN, 5MN and 2MN after induction of tolerancce as shown in Fig. 2.

It has been furthermore shown through the laboratory tests that no relationship between the amount of the measured cyclic guanine mono-phosphate (GMP) and the extent of the inhibitory effect can be observed.

On the basis of these results, it could be speculated that the amount of cyclic GMP increase in peculiar for each nitroderivatives and that mechanisms other that cyclic GMP synthesis are involved.

On the basis of these hypothesis it is possible to explain the differences observed regarding the development of tolerance.

In our experiments, the development of nitrate tolerance seems to be related to cyclic GMP accumulation, being greater in the case of NTG, thus confirming that SH groups consumption is the molecular mechanism responsible for this phenomenon. This reinforce the concept that alternative mechanisms could be operative in the case of the 2MN, which induces a lesser degree of tolerance and a lesser increase of cyclic GMP.

For this reason isosorbide 2-mononitrate can be used for preparing pharmaceutical compositions suitable for chronic form administration in the treatment of angina pectoris.

Isosorbide 2-mononitrate can be also used successfully in preparing compositions suitable for administration by tongue absorption in the acute treatment of angina pectoris.

In this form of administration isosorbide 2-mononitrate acts rapidly at low dosage, has a more persistent effect than nitroglycerin and, in contrast to isosorbide dinitrate, does not develop tolerance.

Detailed description of the invention

The pharmacological characteristics of isosorbide 2-mononitrate and the advantages of its use in the treatment of angina pectoris in its chronic and acute forms will be more apparent from the following detailed description

Isosorbide 2-mononitrate (2MN) was used in a clinical trial conducted on thirteen patients suffering from angina precipitated by steady effort.

The trial comprised evaluating the effectiveness of the 2MN before and after chronic treatment with active doses of 2MN.

For this purpose the patients underwent double blind treatment with 20 mg of 2MN in tablets to be swallowed, and with placebo, they being subjected two hours after taking the preparations to the ergometer bicycle effort test, measurements being made of the exercise time (minutes), maximum tolerated load (watts) and the downward level difference of the S-T curve on the electrocardiogram (mm).

The patients were then treated with 20 mg of 2MN in tablets for oral administration at a posology of three administrations per day for two months, a time well sufficient for tolerance to arise.

During this period all other drugs were suspended except for nitroglycerin for acute attacks.

After two months of chronic treatment the patients were subjected to a further acute double blind test with the ergometer bicycle conducted under the same conditions as the initial test.

The trial results are given in Table 1.

TABLE 1

| Acute effects of 2MN in patients treated chronically with 2MN for two months | | | | | | |
|---|---|---|---|---|---|---|
| | Before chronic treatment After chronic treatment | | | After chronic treatment | | |
| | 2MN | placebo | Δ % | 2MN | placebo | Δ % |
| Exercise time (min) | 8.6±1.4 | 5.5±2.8 | +56 (P<0.001) | 8.9±1.1 | 5.8±0.6 | +53 (P<0.001) |
| Max work tolerated (watt) | 144±36 | 111±23 | +29 (P<0.05) | 148±36 | 114±23 | +36 (P<0.05) |
| S-T (mm) | 1.5±0.7 | 2.1±0.5 | -29 (P<0.05) | 1.8±0.9 | 2.4±0.8 | -25 (P<0.05) |

The data of Table 1 indicate that, compared with the placebo, the 2MN results in considerable increase in the exercise time and maximum work tolerated, whereas it reduces the downward level difference of the S-T curve, the data also demonstrating that the effects of the 2MN are practically equal in the test conducted before the chronic treatment and in the test conducted after the chronic treatment.

This demonstrates that the activity of the 2MN remains unaltered with time and that the 2MN therefore does not give rise to tolerance.

Again, in periodic checks carried out during the chronic treatment there was a noted reduction in anginal attacks, nitroglycerin consumption and arterial pressure, thus demonstrating that the chronic treatment was effected with reliably active doses.

Similar results to those described were obtained in another trial in which tablets for swallowing containing 10 mg of 2MN were administered chronically.

2MN is therefore a drug which solves the problem of chronic treatment of angina pectoris in that it maintains its activity with time, without developing tolerance, by means of a posology acceptable to the patient.

It has also been found that isosorbide 2-mononitrate is also effective in the acute treatment of angina pectoris when administered in compositions for tongue absorption.

When administered in a dose of 5 mg in tablets for tongue absorption to seven patients with ischemic cardiopathy and studied by means of a cardiac catheter, isosorbide 2-mononitrate leads to a reduction in the pulmonary artery pressure.

Activity commences 3-4 minutes after taking the drug, and lasts an average of 80 minutes.

The results obtained are given in Table 2.

TABLE 2

| Acute treatment of patients with isosorbide 2-mononitrate | | | |
|---|---|---|---|
| Patient | Max decrease in pulmonary artery pressure % | Commencement of activity (seconds) | Duration of activity (seconds) |
| 1 | 20 | 180 | 75 |
| 2 | 38 | 250 | 96 |
| 3 | 30 | 230 | 70 |
| 4 | 27 | 160 | 80 |
| 5 | 31 | 200 | 86 |
| 6 | 40 | 210 | 70 |
| 7 | 29 | 200 | 87 |
| $\overline{M}$ | $\overline{30}.7$ | $\overline{204}.2$ | $\overline{80}.6$ |

This activity found confirmation in clinical trials on patients with angina pectoris in which administration by tongue absorption of isosorbide 2-mononitrate was able to prevent anginal attack or to cause its rapid regression. The doses used were 5 mg of 2MN in tablets for tongue absorption administered on onset of the anginal attack.

Analogous results were obtained on administering 2MN as spray solutions for tongue use.

These results demonstrate that isosorbide 2-mononitrate in compositions for tongue absorption constitutes a drug which is very useful in acute therapy of angina pectoris.

The compositions of the invention can be prepared in various forms and preferably in the form of tablets or spray solutions.

The excipients used for the compositions in table form are substances such as carbohydrates, magnesium stearate, talc and finely powdered silica. The preferred carbohydrates are lactose, starch, microcrystalline cellulose, mannitol and calcium carboxymethylcellulose.

These compounds, and particularly lactose and microcrystalline cellulose or calcium carboxymethylcellulose, improve the stability of the isosorbide 2-mononitrate and therefore that of the tablets, by protecting them from any disintegration which might happen on account of the low melting point of the isosorbide 2-mononitrate. It has been ascertained that the loss of active principle in the pharmaceutical forms (i.e. tablets) in the long period is minimal by the use of the above mentioned excipients.

The other excipients provide the appropriate pharmaco-technical characteristics for the specific use of the compositions, and in particular the mannitol and the carboxymethylcellulose provide the characteristics which aid the instant crumbling of tablets for tongue use.

To prepare tablets, the various components are sieved, mixed and compressed to obtain large tablets, which are then ground to obtain a uniform granular product which is used to prepare the final tablets by a second compression step.

The tablets obtained have good organoleptic characteristics, have uniform weight and good resistance to disintegration and can be prepared with doses suitable for swallowing and doses suitable for tongue use.

Thus, swallowing tablets for chronic treatment of angina pectoris can be prepared containing 10 mg or 20 mg of isosorbide 2-mononitrate and tongue tablets for acute treatment of angina pectoris can be prepared containing 5 mg of isosorbide 2- mononitrate.

The compositions of the invention are also prepared as a solution for spray administration.

To prepare such compositions, the isosorbide 2-mononitrate is dissolved in alcohol, preferably ethyl alcohol, and glycerin is added. In this manner a solution is obtained, suitable for filling into bottles for use as a spray for tongue administration. These compositions have numerous advantages such as rapidity and ease of application, elimination of the danger of contamination, instant drug response, and reduced possibility of decomposition of the active substance under conditions of high drug concentration in a restricted area.

The following examples of compositions containing isosorbide 2-mononitrate as active substance for chronic and acute treatment of angina pectoris are given below for the purposes of non-limiting illustration.

EXAMPLE 1

| Tablets containing 20 mg of isosorbide 2-mononitrate | |
| --- | --- |
| Each 210 mg tablet contains: | |
| Isosorbide 2-mononitrate | 20 mg |
| Lactose | 44 mg |
| Starch | 55 mg |
| Avicel pH 102 (microcrystalline cellulose) | 75 mg |
| Magnesium stearate | 6 mg |
| Talc | 4 mg |
| Aerosil 200 | 6 mg |

To prepare the tablets the various components are sieved to remove the agglomerations and are then mixed in a mixer for 30 minutes to obtain a homogeneous mixture.

The mixture is subjected to initial compression in an automatic rotary compression machine, in which the mixture is transformed into large tablets.

These tablets are then ground to obtain a uniform granular product, which is then subjected to final compression using an automatic rotary machine provided with suitable punches.

The tablets obtained have good organoleptic characteristics, weight uniformity and good resistance to disintegration.

EXAMPLE 2

| Tablets containing 10 mg of isosorbide 2-mononitrate | |
| --- | --- |
| Each 210 mg tablet contains: | |
| Isosorbide 2-mononitrate | 10 mg |
| Lactose | 54 mg |
| Starch | 55 mg |
| Avicel pH 102 (microcrystalline cellulose) | 75 mg |
| Magnesium stearate | 6 mg |
| Talc | 4 mg |
| Aerosil 200 | 6 mg |

The tablets are prepared by the procedure described in Example 1, to obtain tablets having the same characteristics as those of Example 1.

EXAMPLE 3

| Tablets containing 5 mg of isosorbide 2-mononitrate for tongue use | |
|---|---|
| Each 80 mg tablet contains: | |
| Isosorbide 2-mononitrate | 5 mg |
| Corn starch | 25 mg |
| Lactose | 25 mg |
| Mannitol | 19 mg |
| Calcium carboxymethylcellulose | 4 mg |
| Magnesium stearate | 2 mg |

The tablets are prepared by the procedure described in Example 1, the pharmaco-technical characteristics of the prepared tablets being such that they instantaneously crumble on administration.

EXAMPLE 4

| Tablets containing 20 mg of isosorbide 2-mononitrate | |
|---|---|
| Each 210 mg tablet contains: | |
| Isosorbide 2-mononitrate | 20 mg |
| Starch | 75 mg |
| Lactose | 75 mg |
| Mannitol | 23 mg |
| Calcium carboxymethylcellulose | 11 mg |
| Mg stearate | 6 mg |

The tablets are prepared by the procedure described in Example 1.

EXAMPLE 5

Isosorbide 2-mononitrate spray

The composition of this example is a solution in an atomizer bottle for tongue use.
Each bottle contains:

| Isosorbide 2-mononitrate | 200 mg |
|---|---|
| Glycerin | 3 g |
| Ethanol | to make up to 20 ml |

To prepare this composition the isosorbide 2-mononitrate is dissolved in 15 ml of ethanol and the glycerin is gradually added. The volume is then made up to 20 ml with further ethanol to obtain a clear solution suitable for use in a spray bottle.

**Claims**

1. Pharmaceutical compositions comprising isosorbide-2-mononitrate for the chronic treatment and acute treatment of angina pectoris.

2. Pharmaceutical compositions comprising isosorbide-2-mononitrate for the chronic treatment of angina pectoris, in the form of tablets containing 10 or 20 mg of active substance and comprising excipients able to stabilize the isosorbide 2-mononitrate.

3. Pharmaceutical compositions according to claim 2, characterised in that said excipients able to stabilize the isosorbide 2-mononitrate are lactose and microcrystalline cellulose or calcium carboxymethylcellulose.

4. Pharmaceutical compositions comprising isosorbide-2-mononitrate for the acute treatment of angina pectoris in the form of tablets for tongue absorption containing 5 mg of active substance and comprising excipients able to stabilize the isosorbide 2-mononitrate, selected from lactose and microcrystalline cellulose or calcium carboxymethylcellulose.

5. Pharmaceutical compositions comprising isosorbide-2-mononitrate for the acute treatment of angina pectoris in the form of alcoholic spray solutions for tongue use.

6. Pharmaceutical compositions according to claim 5, characterised in that said alcoholic solutions are ethanolic solutions comprising glycerin.

7. Pharmaceutical compositions for the chronic treatment of angina pectoris, in the form of a tablet containing 20 mg of isosorbide 2-mononitrate, 44 mg of lactose, 55 mg of starch, 75 mg of microcrystalline cellulose, 6 mg of magnesium stearate, 4 mg of talc and 6 mg of aerosil.

8. Pharmaceutical compositions for the chronic treatment of angina pectoris, in the form of a tablet containing 10 mg of isosorbide 2-mononitrate, 54 mg of lactose, 55 mg of starch, 75 mg of microcrystalline cellulose, 6 mg of magnesium stearate, 4 mg of talc and 6 mg of aerosil.

9. Pharmaceutical compositions for the acute treatment of angina pectoris by tongue use, in the form of a tablet containing 5 mg of isosorbide 2-mononitrate, 25 mg of corn starch, 25 mg of lactose, 19 mg of mannitol, 4 mg of calcium carboxymethylcellulose and 2 mg of magnesium stearate.

10. Pharmaceutical compositions for the chronic treatment of angina pectoris, in the form of a tablet containing 20 mg isosorbide 2-mononitrate, 75 mg starch, 75 mg lactose, 23 mg mannitol, 11 mg Ca-carboxymethylcellulose, 6 mg Mg stearate.

11. Therapeutic chronic treatment of angina pectoris consisting in administering orally isosorbide-2-mononitrate in form of posological units containing 10 or 20 mg of active substance.

FIG. 1

NTG $10^{-7}$M

NTG $10^{-7}$M

0,5 g

NOR $10^{-6}$M

NOR $10^{-6}$M

2h incubation
with NTG $10^{-7}$M

5 min

FIG. 2

EP 0 425 902 A1

FIG. 3

EP 0 425 902 A1

European Patent Office

**PARTIAL EUROPEAN SEARCH REPORT**
which under Rule 45 of the European Patent Convention
shall be considered, for the purposes of subsequent
proceedings, as the European search report

Application number

EP 90 11 9954

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | MED. WELT, vol. 32, no. 14a, 1981, pages 524-526; N. REIFART et al.: "Vergleich der antianginösen Wirksamkeit und Wirkdauer von oral verabreichtem Isosorbiddinitrat (ISDN), Isosorbid-2Mononitrat (IS-2-MN) und Isosorbid-5-Mononitrat (IS-5-MN)" <br><br> * Whole document, especially page 524" <br><br> -- | 1-4, 7-10 | A 61 K 31/34 |
| X | Z. KARDIOL., vol. 68, no. 10, 1979, pages 687-693, Dr. Dietrich Steinkopff Verlag, Darmstadt, DE; M. STAUCH et al.: "Die Wirkung von Isosorbiddinitrat, Isosorid-2- und 5-Mononitrat auf das Belastungs-Ekg und auf die Hämodynamik während Vorhofstimulation bei Patienten mit Angina pectoris" <br><br> ./. | | **TECHNICAL FIELDS SEARCHED (Int. Cl.4)** <br><br> A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 11

Reason for the limitation of the search:

Method for treatment of the human or animal body by surgery or therapy (See art. 52(4) of the European Patent Convention)

| Place of search | Date of completion of the search | Examiner |
|---|---|---|
| THE HAGUE | 15-11-1990 | KLAVER |

| Category | Citation of document with indication, where appropriate, of relevant passages | Relevant to claim | CLASSIFICATION OF THE APPLICATION (Int. Cl.4) |
|---|---|---|---|
| | * Whole document, especially page 691: "Diskussion" * | 1-4, 7-10 | |
| X | ARZNEIMITTEL FORSCHUNG, vol. 33(II), no. 8, 1983, pages 1117-1121; H. LAUFEN et al.: "Vergleichende antianginöse, hämodynamische und pharmakokinetische Wirkung von Isosorbid-2-mononitrat und Isosorbiddinitrat an der Ratte" | | |
| | * Whole document * | 1-4, 7-10 | |
| X | EP-A-0 288 895 (KALI-CHEMIE) | | TECHNICAL FIELDS SEARCHED (Int. Cl.4) |
| | * Claims ; especially claim 7 * | 1,5,6 | |
| A | DE-A-3 619 083 (J.W. NICOLAI) | | |
| | * Claims 1-8 * | 1-10 | |
| A | EP-A-0 219 161 (EURAND ITALIA) | | |
| | * Claims * | 1-10 | |
| A | DE-A-3 325 652 (RENTSCHLER GmbH) | | |
| | * Claims * | 1-10 | |
| A | US-A-3 886 186 (W. DVONCA et al.) | | |
| | * Whole document * | 1-10 | |

----

EPO Form 1505.3 06.78